# EUROPEAN PATENT SPECIFICATION

(11) **EP 2 756 097 B1**
(45) Date of publication and mention of the grant of the patent: **02.03.2022**
(21) Application number: 12740380.6
(22) Date of filing: 30.07.2012
(51) Int. Cl.: C12Q 1/68, C12Q 1/18

(54) **METHOD FOR DETECTING ANTIBIOTIC RESISTANCE**
VERFAHREN ZUR ERKENNUNG EINER ANTIBIOTIKARESISTENZ
PROCÉDÉ POUR DÉTECTER UNE RÉSISTANCE AUX ANTIBIOTIQUES

(30) Priority: 29.07.2011 EP 11176027
(43) Date of publication of application: 23.07.2014
(73) Proprietor: MetaSystems Indigo GmbH, 68804 Altlußheim (DE)
(72) Inventor: STANGE, Mirko, 40213 Düsseldorf (DE); STEIN, FREIHERR VON, Walter, 40474 Düsseldorf (DE)
(74) Representative: Remus, Alvaro Johannes
(86) International application number: PCT/EP2012/064904
(87) International publication number: WO 2013/017573

(56) References cited:
- WO-A1-2010/135480
- WO-A2-2010/129532
- WO-A2-2011/087789
- SANTISO REBECA ET AL: "A rapid in situ procedure for determination of bacterial susceptibility or resistance to antibiotics that inhibit peptidoglycan biosynthesis", BMC MICROBIOLOGY, vol. 11, August 2011 (2011-08), XP002686563,
- HIGGINS DEBORAH L ET AL: "Telavancin, a multifunctional lipoglycopeptide, disrupts both cell wall synthesis and cell membrane integrity in methicillin-resistant Staphylococcus aureus", ANTIMICROBIAL AGENTS AND CHEMOTHERAPY, vol. 49, no. 3, March 2005 (2005-03), pages 1127-1134, XP002686564, ISSN: 0066-4804
- MCCALLUM N ET AL: "Strain dependence of the cell wall-damage induced stimulon in Staphylococcus aureus", BIOCHIMICA ET BIOPHYSICA ACTA - GENERAL SUBJECTS, ELSEVIER SCIENCE PUBLISHERS, NL, vol. 1760, no. 10, 1 October 2006 (2006-10-01), pages 1475-1481, XP025014988, ISSN: 0304-4165, DOI: 10.1016/J.BBAGEN.2006.06.008 [retrieved on 2006-10-01]

### SEQUENCE LISTING

<110> miacom Diagnostics GmbH
<120> Method for detecting antibiotic resistance
<130> 11254WO
<150> EP11176027.8
   <151> 2011-07-29
<160> 2
<170> PatentIn version 3.5
<210> 1
   <211> 27
   <212> DNA
   <213> Artificial Sequence
<220>
   <223> Molecular beacon as probe for Staphylococcus aureus
<400> 1
   cctgcaagct tctcgtccgt tcgcagg 27
<210> 2
   <211> 28
   <212> DNA
   <213> Artificial Sequence
<220>
   <223> Molecular beacon as probe for Staphylococcus spp.
<400> 2
   ccaactttcg cacatcagcg tcagttgg 28

## Description

### Background of the invention

The invention relates to a method for detecting resistance of *Staphylococcus aureus* cells to methicillin or vancomycin in a sample, wherein said cells are treated with lysostaphin, and wherein said cells are identified by at least one nucleic acid molecule comprising a marker component and being capable of hybridising with a target nucleic acid sequence of the *Staphylococcus aureus* cells.

### Prior art

*Staphylococcus aureus* (*S*. *aureus*) is one of the most common causes of nosocomial or community-based infections, leading to serious illnesses with high rates of morbidity and mortality. In recent years, the increase in the number of bacterial strains that show resistance to the antibiotic methicillin (methicillin-resistant *Staphylococcus aureus* = MRSA) has become a serious clinical and epidemiological problem because this antibiotic or analogues thereof are considered the first option in the treatment of Staphylococci infections. The resistance to this antibiotic implies resistance to all β-lactam antibiotics. For these reasons, accuracy and promptness in the detection of methicillin resistance is of key importance to ensure correct antibiotic treatment of infected patients as well as control of MRSA isolates in hospital environments, so that spreading of MRSA can be avoided. MRSA strains harbour the mecA gene, which encodes a modified PBP2 protein (PBP2<1> or PBP2a) with low affinity for methicillin and all β-lactam antibiotics. Phenotypic expression of methicillin resistance may alter depending on the growth conditions for *S*. *aureus,* such as temperature or osmolarity of the medium, and this may affect the accuracy of the methods used to detect methicillin resistance (1). Hetero-resistant bacterial strains may evolve into fully resistant strains and therefore be selected in those patients receiving β-lactam antibiotics, thus causing therapeutic failure. From a clinical point of view, they should therefore be considered fully resistant.

There are several methods for detecting methicillin resistance (2, 3) including classical methods for determining a minimum inhibitory concentration MIC (disc diffusion, Etest, or broth dilution), screening techniques with solids culture medium containing oxacillin, and methods that detect the mecA gene or its protein product (PBP2<1> protein) (4, 5). Detection of the mecA gene is considered as the reference method for determining resistance to methicillin (2). However, many laboratories throughout the world do not have the funds required, the capacity or the experienced staff required to provide molecular assays for detecting MRSA isolates. It is therefore essential that other, more useful, screening methods are incorporated into routine clinical practice. Moreover, the presence of antibiotic resistance has it's relevance at several levels, all of which are of clinical significance:
1. Presence of a gene conveying resistance, such as mecA, mef(E),
2. Presence of a repressor gene inhibiting the phenotypic expression of said resistance mechanism, e.g. MecA repressor,
3. Multiple resistance mechanisms, e.g. Macrolide resistance via modification of the ribosomal binding site and presence of efflux mechanism(s), and
4. Level of expression of said resistance mechanism regulated via transcription and translation detectable as the phenotype.

Current cultural techniques require the isolation of a discrete colony and the subsequent identification and resistance testing, assuming that a single colony is derived from a single cell and is therefore deemed to be pure. In reality however, the generation of a pure colony from a clinical sample, where pathogens frequently live in bio-film communities, cannot be guaranteed. Equally, using amplification technologies, nucleic acid sequences from multiple cells are extracted and amplified and can therefore render false positive results. Only if identification and resistance can be performed and be read on individual cells, is it possible to obtain a true picture of the invading pathogen.

A wide range of antibiotics carry a primary amino group. It is well known in the art that reagents such as Fluoresceinisothiocyanate (FITC), Fluorecein- N-hydroxysuccinimide ester will readily react with said primary amines.

The increasing spread of antibiotic resistance in both community and healthcare systems necessitates the precision and speed of molecular biology. However, the complexity and cost of these assays prohibits the widespread application in a routine testing environment.

The characterisation of micro-organisms in routine diagnostic procedures encompasses the determination of a species' identity and its sensitivity towards antibiotics. In order to achieve this, micro-organisms need to be taken from their environment and enriched in a selective environment for the separate identification (ID) and antibiotic sensitivity testing (AST). Currently the AST/ID of micro-organisms is achieved by identifying presence or absence of an array of biochemical features and the (non-) capability to grow in the presence of antibiotics. Alternatively DNA can be extracted from a sample and the then pooled DNA is tested for the presence/absence of specific sequences utilising gene amplification techniques. This can signal the presence of an organism in the sample. Equally, the presence of a gene coding for antibiotic resistance in the sample can be detected. By definition, extracting DNA directly from a sample renders pooled DNA from an unknown mixture of cells. Unequivocal results can only be achieved if the DNA is extracted from a pure colony.

WO 2010/129532 A2 discloses diagnostic methods and devices for determining antibiotic susceptibility of bacteria in a sample. The diagnostic methods are based on the observation that the application of shear stress and/or chemical stress to bacteria in the presence of an antibiotic permits one to determine the sensitivity or resistance of a bacterium to the antibiotic without requiring a cell growth phase of the bacterium. Shear and/or chemical stress applied to bacteria catalyzes the biochemical pathways to repair damage to the cells. These pathways are the targets of antibiotics and therefore repair is inhibited in the presence of the antibiotic. In general, bacteria that are susceptible to such an antibiotic will die in the presence of a stressor, while resistant strains can repair the stress-induced damage. Thus, the susceptibility of the organism can be determined without waiting for bacterial growth.

WO 2011/087789 A2 discloses a method for the detection of the presence or absence of one or more microorganisms in a sample. The method deploys a 4 plurality of probe sets to detect a plurality of microorganisms. The probes in the probe set are detectably labeled. At least one probe set has probes labeled with a combination of detectable labels. The number of detectable labels used in the plurality of probe sets numbers less than the number of microorganisms being detected by the probe set.

WO 2010/135480 A1 discloses a method for the whole-cell analysis of gram-positive bacteria. The method is capable of making a determination of whether or not a sample comprises one or more gram-positive bacteria as well as whether or not some or all of said gram-positive bacteria in said sample possess a selected trait of interest. The method can be used to determine methicillin-resistant staphylococcus aureus, coagulase-negative staphylococci and/or methicillin-sensitive staphylococcus aureus in said sample. The whole-cell analysis can be performed by in-situ hybridization, fluorescence in-situ hybridization, immunocytochemistry, or any combination thereof.

Taking into account the difficulties in identifying a micro-organism and its potential resistance against an antibiotic in a biological sample, it is desirable to be able to quickly identify a pathogen directly from a sample without culturing and without amplification and in addition to be able to detect or exclude the presence of resistance towards an antibiotic of choice.

### Summary of the invention

The object is achieved by a method wherein a first aliquot of the sample is treated with a buffer comprising a low concentration of lysostaphin in the range from 1 to 30 U/ml and a second aliquot of the sample is treated with a buffer comprising a high concentration of lysostaphin in the range from 50 to 500 U/ml, wherein the presence of methicillin-resistant *Staphylococcus aureus (MRSA)* cells is indicated if the marker component is detected within almost all cells in the first aliquot and almost no marked cells are observed in the second aliquot. Surprisingly, it turned out that the cell wall of resistant strains is weakened so that treatment with a disrupting agent in low concentrations results in partial cell wall destruction. Due to cell wall destruction, marker substances can easily enter the cells so that they can be detected by standard methods. This effect is not observed with strains that are sensitive to the antibiotic so that, depending on the concentration of the disrupting agent, a clear distinction between resistant and sensitive cells can be accomplished. The method according to the invention reduces the complexity of resistance testing assays so that an unambiguous assignment of a resistence can be made for individual cells. The method is designed to reduce handling and turnaround time of an assay in order to enable screening programmes such as the screening of all incoming patients for, e.g., MRSA.

For high specificity of the method, the disrupting agent is lysostaphin. Lysostaphin, a zinc metalloendopeptidase extracted from *Staphylococcus simulans,* can lyse *S*. *aureus* by disrupting its peptidoglycan layer. Thus, it can function as an antimicrobial against *Staphylococcus aureus* (1). The gene for lysostaphin has been successfully cloned and expressed in *Bacillus sphaericus* and *Escherichia coli.* The major substrate for lysostaphin is the staphylococcal cell wall, specifically the pentaglycine bridge found in the cell wall of *S*. *aureus.* The use of lysostaphin for chemotherapy was proposed over 30 years ago. Lysostaphin purified from *S*. *simulans* was found to be effective in treating experimental staphylococcal infections.

According to the invention at least two different concentrations of said disrupting agent are used, i.e. a low concentration and a high concentration of the disrupting agent. In this respect, "low concentration" refers to very low concentrations, i.e. concentrations in the range from 1 to 30 U/ml. "High concentration" refers to concentrations higher than "low concentrations", i.e concentrations in the range from 50 to 500 U/ml. Using high and low concentrations of the disrupting agent within one assay allows for more precise differentiation between sensitive and resistant Staphylococcus aureus For instance, if only few disrupting agent is present ("low concentration"), the cell wall of the Staphylococcus aureus is not or only minimally affected. In case of sensitive cells, marker substances cannot enter the cells and only some cells are stained. In case of resistant cells, almost all cells are marked as the marker substances can enter the cells through the disrupted cell wall. If higher amounts of disrupting agent are present ("high concentration"), the cell wall of the Staphylococcus aureus is sufficiently affected so that the marker substances can even enter the sensitive cells and almost all cells are marked. In contrast, resistant cells are completely destroyed, i.e. the cells burst and almost no marked cells can be observed.

Advantageously, detection of at least one damaged cell indicates the presence of a resistance to the antibiotic substance and/or detection of at least one undamaged cell indicates the presence of sensitivity to the antibiotic substance.

In a particularly preferred embodiment of the invention, said *Staphylococcus aureus* cells are incubated with an inductor substance, preferably an antibiotic, before they are treated with lysostaphin. This preincubation advantageously enhances the sensitivity of the method according to the invention.

It is another advantage of the invention that the method according to the invention can be performed with a blood sample presumably comprising said *Staphylococcus aureus.* Using whole blood directly from the patient renders the assay easier and faster as complex and time-consuming processing of the patient's sample before performing the test for antibiotic resistance is avoided.

For simultaneous identification of Staphylococcus aureus and detection of resistance within the same assay, the Staphylococcus aureus is subsequently or concomitantly identified by at least one nucleic acid molecule comprising a marker component and being capable of hybridising with a target nucleic acid sequence of the *Staphylococcus aureus* cells. According to the invention, the nucleic acid molecule is a molecular beacon comprising a fluorescent group.

The invention is further described in detail with reference to the figure and examples.

### Brief description of the figure

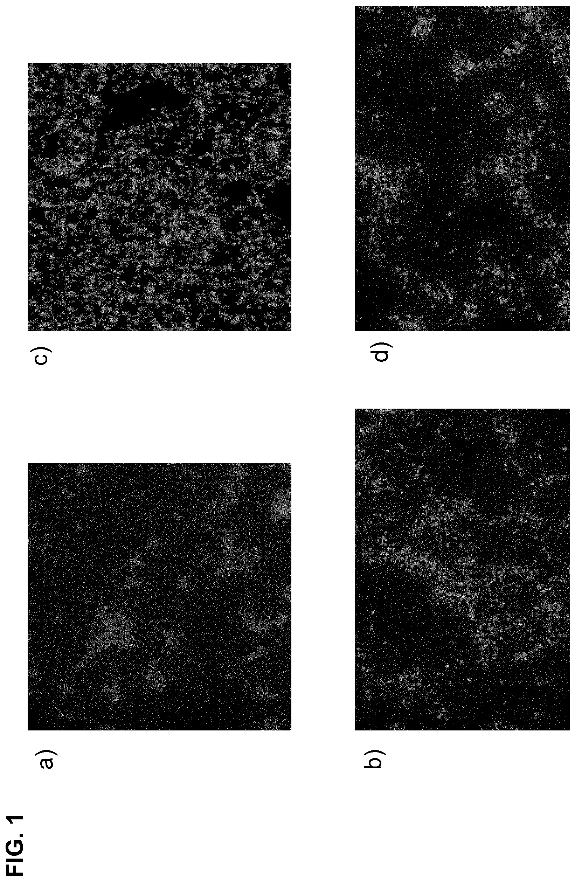

Figure 1 shows fluorescence microscopic photographs of samples comprising methicillin-sensitive *Staphylococcus aureus* (MSSA) or methicillin-resistant *Staphylococcus aureus* (MRSA), Probe = Red-marked beacon specific for *Staphylococcus aureus*:
a) MSSA, Lysis Buffer comprising 25 U/ml lysostaphin.
b) MRSA, Lysis Buffer comprising 25 U/ml lysostaphin.
c) MSSA, Lysis Buffer comprising 200 U/ml lysostaphin.
d) MRSA, Lysis Buffer comprising 200 U/ml lysostaphin.

### Description of examplary and preferred embodiments of the invention

According to a preferred embodiment of the invention, low concentrations of Lysostaphin are used to perforate the cell wall of Staphylococcus aureus. Depending on the concentration of the enzyme, easily detectable differences in cell staining can be observed. In case of MRSA the cell wall is weakened, probably because the modified PBP2a cannot completely compensate the missing regular PBP. The teatment with Lysostaphin destroys the resistant cells (MRSA), which are then not microscopically visible anymore, while sensitive cells (MSSA) seem to be morphologically unaffected under the same conditions.

According to another preferred embodiment of the invention, molecular beacons comprising a fluorescent group are used as marker substance for detecting resistant cells. The molecular marker as used according to the invention is descibed in WO-A-2008/043543 The marker substance comprises a nucleic acid beacon capable of forming a hybrid with a target nucleic acid sequence and capable of forming a stem-loop structure if no hybrid is formed with the target sequence. The beacon comprises a nucleic acid portion comprising a sequence complementary to the target nucleic acid sequence and a pair of two complementary sequences capable of forming a stem. In particular, the two complementary sequences are flanking the specific sequence, i.e. a first sequence is attached at the 3' end of the specific sequence and a second sequence complementary to the firct sequence is attached at the 5' end of the specific sequence.The marker substance further comprises an effector and an inhibitor, wherein the inhibitor inhibits the effector when the nucleic acid forms a stem-loop structure, and wherein the effector is active when the nucleic acid is not forming a stem-loop structure. The effector may be attached at one of the two complementary sequences capable of forming a stem, whereas the inhibitor may be attached at the other of the two complementary sequences so that the inhibitor essentially inhibits the effector activity when a stem is formed, and that the effector is active when the hairpin is open. Preferably, the effector is attached at the 5' end or the 3' end of the beacon, respectively, or at a position which is 1, 2, 3, 4, or 5 nucleotides distant to the 5' end or the 3' end, respectively. The inhibitor is preferably attached at the other end not covered by the effector, i.e. at the 3' end or the 5' end, respectively, or at a position which is 1, 2, 3, 4, or 5 nucleotides distant to the 3' end or the 5' end, respectively. Hybridisation of the beacon with a target sequence, preferably a rRNA sequence of the microorganism, may take place under conditions where the loop is open. A beacon which is not forming a stem when hybridizing is capable of annealing to a target rRNA sequence, for instance, and can therefore achieve successful hybridisation. This molecular beacon is coupled to a fluorescent group and therefore suitable for in-situ hybridisation, in particular FISH.

Very low concentrations of Lysostaphin are sufficient to perforate resistant cells such that the beacons can enter the cells and lead to a significant fluorescent signal, while sensitive cells are not perforated and thus not stained (about 2 % of the cells are always stained, probably because they are dividing). For example, 4 aliquots of a sample obtained from the patient to be tested are placed on separate fields on a suitable substrate, preferably a microscope slide. The fields are loaded as set forth:
Field 1 - Positive control: Unspecific red-marked beacon (detects all bacteria), lysis buffer without Lysostaphin (or very low concentration, e.g. 25 Units/ml);
Field 2 - Red-marked beacon specific for *Staphylococcus ssp.* and green-marked beacon specific for *Staphylococcus aureus,* lysis buffer like field 1;
Field 3 - Beacon like field 1, lysis buffer with Lysostaphin (between 50 and 250 Units pro ml);
Field 4 - Beacon like field 2, lysis buffer like field 3.

Sequences of the beacons:
*Staphylococcus aureus*: CCTGCAAGCTTCTCGTCCGTTCGCAGG (SEQ ID NO:1)
*Staphylococcus spp*.: CCAACTTTCGCACATCAGCGTCAGTTGG (SEQ ID NO:2)

The lysis buffer according to the invention may comprise:

| | |
|---|---|
| 2 mM | Tris-HCI, pH 8.3, |
| 25-250 U/ml | Lysostaphin (*S.simulans,* expressed in *E.coli*; Sigma-Aldrich), |
| 50,000 U/ml | Lysozyme (human milk, expressed in rice; Sigma-Aldrich). |

In fields 1 and 2 no or only few Lysostaphin is present so that the cell wall is not (or only minimally) affected. In case of MSSA, the beacons cannot enter the cells and only some cells are stained. In case of MRSA, almost all cells are stained as the beacons can enter the cells through the disrupted cell wall.

In fields 3 and 4 stronger lysis buffer is present and, therefore, these fields allow for differenciation between coagulase-negative Staphylococci and *Staphylococcus areus.* In case of MSSA, the cells wall is sufficiently affected so that the beacons can enter the cells and almost all cells are stained. In contrast, MRSA cells are completely destroyed, i.e. the cells burst and almost no stained cells can be observed. In this case, cord-like shining due to free rRNA that was released from the disrupted cells occurs occasionally (background shining).

Consequently, if fields 1 and 2 are shining more than fields 3 and 4, MRSA cells are present. If fields 3 and 4 are shining more than fields 1 and 2, MSSA cells are present. Accordingly, analysis of the assay is fast and reliable and the resistance of the cells to an antibiotic can be easily detected. Moreover, as molecuar beacons specific to the rRNA of different microorganism are used as marker substance, the microorganisms can be simultaneously identified within the same assay.

Preferably, the patient's sample is preincubated with an inductor substance in order to induce the resistance mechanism in the microorganism. It is assumed that the induction leads to reorganization of the microorganism's membrane structure. For example, 6 ml of a blood sample are incubated with 30 µg Cefotaxim for 30 minutes before the assay is performed as described above. Preincubation with an antibiotic significantly enhances sensitivity of the method according to the invention. However, the invention is not limited to the use of Cefotaxim as an inductor substance. Other antibiotics or substances having similar effect may be used as well.

### EXAMPLE:

**Figure 1** shows fluorescence microscopic photographs of patient's samples detected with red-marked beacons specific for *Staphylococcus aureus.* With weak lysis buffer sensitive cells (MSSA) are not perforated and thus not significantly stained (**a**), while this low concentration of Lysostaphin is sufficient to perforate resistant cells (MRSA) such that the beacons can enter the cells and lead to a significant fluorescent signal (**b**). It becomes apparent from Figure 1 (b) that some cells are always stained, probably because they are dividing. With stronger lysis buffer the cell wall of sensitive cells (MSSA) is sufficiently affected so that the beacons can enter the cells and almost all cells are stained (**c**). In contrast, resistant cells (MRSA) are completely destroyed, i.e. the cells burst and almost no stained cells can be observed (**d**). In the last case, cord-like shining due to free rRNA that was released from the disrupted cells occurs (background shining).

### Non-patent literature

1. Kokai-Kun JF, Walsh SM, Chanturiya T, Mond JJ (2003). "Lysostaphin cream eradicates Staphylococcus aureus nasal colonization in a cotton rat model". Antimicrob. Agents Chemother. 47 (5): 1589-97.
2. Chambers, H. F. (1997). Methicillin resistance in staphylococci: molecular and biochemical basis and clinical implications. Clinical Microbiology Reviews 10, 781-791.
3. Louie, L., Matsumura, S. O., Choi, E. et al. (2000). Evaluation of three rapid methods for detection of methicillin resistance in S. aureus. Journal of Clinical Microbiology 38, 2170-3.
4. Van Leeuwen, W. B., Van Pelt, C, Luijendijk, A. et al. (1999). Rapid detection of methicillin resistance in Staphylococcus aureus isolates by the MRSA-screen latex agglutination test. Journal of Clinical Microbiology 37, 3029-30.
5. Louie, L., Majury, A., Goodfellow, J. et al. (2001 ). Evaluation of a latex agglutination test (MRSA-Screen) for detection of oxacillin resistance in coagulase-negative staphylococci. Journal of Clinical Microbiology 39, 4149-51.

## Claims

1. Method for detecting resistance of *Staphylococcus aureus* cells to methicillin or vancomycin in a sample, wherein said cells are treated with lysostaphin, and wherein said cells are identified by at least one nucleic acid molecule comprising a marker component and being capable of hybridising with a target nucleic acid sequence of the *Staphylococcus aureus* cells, **characterized in that** a first aliquot of the sample is treated with a buffer comprising a low concentration of lysostaphin in the range from 1 to 30 U/ml and a second aliquot of the sample is treated with a buffer comprising a high concentration of lysostaphin in the range from 50 to 500 U/ml, wherein the presence of methicillin-resistant *Staphylococcus aureus (MRSA)* cells is indicated if the marker component is detected within almost all cells in the first aliquot and almost no marked cells are observed in the second aliquot.

2. Method according to claim 1, wherein said *Staphylococcus aureus* cells are incubated with an inductor substance, preferably an antibiotic, before they are treated with lysostaphin.

3. Method according to claim 1 or 2, wherein said detection of the resistance is performed with a blood sample presumably comprising said *Staphylococcus aureus.*

4. Method according to any one of the preceding claims, wherein said nucleic acid molecule is a molecular beacon comprising a fluorescent group.

## Patentansprüche

1. Verfahren zum Nachweis der Resistenz von *Staphylococcus aureus* - Zellen gegen Methicillin oder Vancomycin in einer Probe, wobei diese Zellen mit Lysostaphin behandelt werden, und wobei diese Zellen mittels mindestens eines Nukleinsäuremoleküls identifiziert werden, das einen Markerbestandteil umfasst und in der Lage ist, mit einer Ziel-Nukleinsäuresequenz der *Staphylococcus aureus* - Zellen zu hybridisieren, **dadurch gekennzeichnet, dass** ein erstes Aliquot der Probe mit einem Puffer behandelt wird, der eine geringe Lysostaphin-Konzentration im Bereich von 1 bis 30 U/ml umfasst, und ein zweites Aliquot der Probe mit einem Puffer behandelt wird, der eine hohe Lysostaphin-Konzentration im Bereich von 50 bis 500 U/ml umfasst, wobei das Vorliegen von Methicillin-resistenten *Staphylococcus aureus (MRSA)* - Zellen erkennbar ist, wenn der Markerbestandteil in nahezu allen Zellen im ersten Aliquot detektiert wird und nahezu keine markierten Zellen im zweiten Aliquot wahrnehmbar sind.

2. Verfahren nach Anspruch 1, wobei die *Staphylococcus aureus* - Zellen mit einer Induktor-Substanz inkubiert werden, vorzugsweise einem Antibiotikum, bevor sie mit Lysostaphin behandelt werden.

3. Verfahren nach Anspruch 1 oder 2, wobei der Nachweis der Resistenz mit einer Blutprobe, die mutmaßlich *Staphylococcus aureus* umfasst, durchgeführt wird.

4. Verfahren nach einem der vorhergehenden Ansprüche, wobei das Nukleinsäuremolekül ein molekulares Beacon ist, das eine fluoreszierende Gruppe umfasst.

## Revendications

1. Procédé de détection de la résistance de *Staphylococcus aureus* à la méthicilline ou à la vancomycine dans un échantillon, dans lequel lesdites cellules sont traitées avec de la lysostaphine, et dans lequel lesdites cellules sont identifiées par au moins une molécule d'acide nucléique comprenant un composant marqueur et étant capable de s'hybrider avec une séquence d'acide nucléique cible des cellules de *Staphylococcus aureus,* **caractérisé en ce qu'**une première aliquote de l'échantillon est traitée avec un tampon comprenant une faible concentration de lysostaphine dans la plage de 1 à 30 U/ml et une seconde aliquote de l'échantillon est traitée avec un tampon comprenant une concentration élevée de lysostaphine dans la plage de 50 à 500 U/ml, dans lequel la présence de cellules de *Staphylococcus aureus* résistant à la méthicilline (*MRSA*) est indiquée si le composant marqueur est détecté dans pratiquement toutes les cellules dans la première aliquote et que pratiquement aucune cellule marquée n'est observée dans la seconde aliquote.

2. Procédé selon la revendication 1, dans lequel lesdites cellules de *Staphylococcus aureus* sont mises à incuber avec une substance inductrice, de préférence un antibiotique, avant d'être traitées avec de la lysostaphine.

3. Procédé selon la revendication 1 ou 2, dans lequel ladite détection de la résistance est réalisée avec un échantillon de sang comprenant probablement ledit *Staphylococcus aureus.*

4. Procédé selon l'une quelconque des revendications précédentes, dans lequel ladite molécule d'acide nucléique est une balise moléculaire comprenant un groupe fluorescent.
